# EUROPÄISCHE PATENTSCHRIFT

(11) **EP 1 318 946 B1**
(45) Veröffentlichungstag und Bekanntmachung des Hinweises auf die Patenterteilung: **11.08.2004**
(21) Anmeldenummer: 01985247.4
(22) Anmeldetag: 17.09.2001
(51) Int. Cl.: B65D 21/02

(54) **KORB, INSBESONDERE STERILISATIONS-, TRANSPORT- UND/ODER LAGERKORB**
BASKET, IN PARTICULAR A STERILISATION, TRANSPORT AND/OR STORAGE BASKET
PANIER, EN PARTICULIER PANIER DE STERILISATION, DE TRANSPORT ET / OU DE RANGEMENT

(30) Priorität: 21.09.2000 DE 10046757
(43) Veröffentlichungstag der Anmeldung: 18.06.2003
(73) Patentinhaber: Metallverarbeitung Kögel GmbH, 75038 Oberderdingen (DE)
(72) Erfinder: KÖGEL, Rolf-Dieter, 75038 Oberderdingen (DE); SERVAY, Gerd, 75057 Kürnbach (DE)
(74) Vertreter: Bulling, Alexander, Dr.
(86) Internationale Anmeldenummer: PCT/EP2001/010703
(87) Internationale Veröffentlichungsnummer: WO 2002/024535

(56) Entgegenhaltungen:
- DE-U- 9 206 319
- FR-A- 1 449 002
- FR-A- 2 510 067
- US-A- 2 660 328
- US-A- 3 939 981
- US-A- 4 744 474

## Beschreibung

Die Erfindung betrifft einen Korb, insbesondere einen Sterilisations-, Transport- und/oder Lagerkorb mit einem Boden und mit Seitenwänden. Der Boden sowie die Seitenwände sind vorzugsweise aus miteinander verschweißten oder verlöteten Drähten und bilden ein Drahtgitter.

Die Dokumente US 2 660 328 A, US 3 939 981 A, FR 81 14149 A und FR 1449002 A offenbaren solcke Körbe.

Derartige Körbe finden vielseitig in insbesondere Krankenhäusern, Kliniken, Sanatorien oder auch Labors zur Sterilisation, zur Lagerung, zum Transport und zur Organisation von entsprechenden Gütern Verwendung. So werden die Güter nach deren Gebrauch in solchen Körben gesammelt, transportiert, in einem Sterilisationsbecken gereinigt und gegebenenfalls gelagert. Die dafür vorgesehenen Körbe müssen hierbei entsprechenden Anforderungen gerecht werden.

Weiterhin sollen die genannten Körbe zum Einen funktionssicher aufeinander gestapelt werden können. Dabei soll es möglich sein, in dem Korb entsprechende Güter zu lagern oder auch zu transportieren. Weiterhin sollen die Körbe ineinander stapelbar sein, um bei Nichtbenutzung möglichst platzsparend gelagert werden zu können.

Der vorliegenden Erfindung liegt deshalb die Aufgabe zugrunde, Körbe bereitzustellen, die den genannten Anforderungen gerecht werden.

Diese Aufgabe wird bei einem Korb gemäß des Anspruchs 1 gelöst.

Ein derartiger, erfindungsgemäßer Korb hat insbesondere den Vorteil, dass aufgrund des definierten Zusammenwirkens von Haken des einen und Ösen des anderen Korbes ein funktionssicheres Aufeinanderstapeln von mehreren Körben gewährleistet werden kann.

Bei der Erfindung sind die Ösen und Haken derart angeordnet, dass beim Drehen des Korbes um die senkrechte Mittelachse um 180° die Haken des oberen Korbes nicht an oder in den Ösen des unteren Korbes zum Aufliegen kommen und die Körbe ineinander stapelbar sind. Bei derartigen Körben wird neben einem funktionssicheren Stapeln von mehreren Körben ein platzsparendes und günstiges Ineinanderstapeln der Körbe auf einfache Art und Weise, nämlich durch Drehen eines Korbes um die senkrechte Mittelachse um 180°, erreicht.

Vorteilhafterweise kann hierbei vorgesehen sein, dass der Korb im Bereich der Oberkanten von zwei gegenüberliegenden Seitenwänden jeweils zwei Ösen aufweist, wobei der Abstand a der beiden Ösen zueinander an der einen Seitenwand größer als der Abstand b der beiden Ösen an der gegenüberliegenden Seitenwand ist, und dass der Korb im Bereich der Unterkanten der entsprechenden Seitenwänden jeweils zwei Haken aufweist, wobei der Abstand a der beiden Haken zueinander an der einen Seitenwand größer ist als der Abstand b der beiden Haken an der gegenüberliegenden Seitenwand und wobei die Abstände der Haken zueinander den Abständen der Ösen zueinander der gleichen Seitenwand entsprechen. Aufgrund einer derartigen Anordnung der Haken und Ösen kann erreicht werden, dass zwei Körbe einerseits funtkionssicher aufeinander gestapelt werden und andererseits, nach Drehen eines Korbes um 180° um die senkrechte Mittelachse die beiden Körbe ineinander gestapelt werden können. Beim Ineinanderstapeln liegen die Haken des oberen Korbes nicht in oder an den Ösen des unteren Korbes auf, sondern sind seitlich versetzt angeordnet.

Bei einer besonders vorteilhaften Ausbildung der Erfindung sind die Ösen der Korbinnenseite zugewandt. Hierbei kann insbesondere gewährleistet werden, dass die Oberkante des Korbes keine Ausformungen in Form von Ösen aufweist, die in Richtung der Korbaußenseite abstehen. An derartigen Ösen ist eine Verletzungsgefahr nicht gegeben.

Vorteilhafterweise können die erfindungsgemäßen Körbe derart ausgebildet sein, dass die Haken den Oberkanten der Seitenwände zu- oder abgewandt sind. Bei Haken, die den Oberkanten zugewandt sind, können die Haken auf den Ösen zum Aufliegen kommen. Bei Haken, die den Oberkanten abgewandt sind, ist es denkbar, dass die Haken bei aufeinander gestapelten Körben in die Ösen des unteren Korbes eingreifen.

Bei einer besonders bevorzugten Ausführungsform der Erfindung weist der Korb an der Oberkante der Seitenwände zwei weitgehend parallel nebeneinander verlaufende Rahmenelemente aus Draht auf, wobei die Ösen von Verformungen des einen, vorzugsweise des innenliegenden Rahmenelements gebildet werden. Eine derartige Ausführungsform der Erfindung hat den Vorteil, dass die Oberkante des Korbes relativ stabil ausgebildet ist. Außerdem sind keine scharfen Kanten, die zu Verletzungen führen können, vorhanden.

Hierbei kann vorteilhafterweise vorgesehen sein, dass der Boden als auch die Seitenwände von einem zusammenhängenden Drahtgitter gebildet werden, wobei freie Enden der das Drahtgitter bildenden Drähte zwischen den beiden Rahmenelementen enden. Die freien Enden der Drähte sind hierbei nicht zugänglich, weshalb eine Verletzungsgefahr an den freien Enden der Drähte ausgeschlossen ist.

Eine weitere Ausführungsform der Erfindung zeichnet sich dadurch aus, dass der Korb an der Unterkante der Seitenwände ein Bodenrahmenelement aus Draht aufweist, wobei die Haken von Verformungen des Bodenrahmenelements gebildet werden. Aufgrund einer einstückigen Ausbildung des Bodenrahmenelments und der Haken entfallen zusätzliche Bauteile, die die Haken bilden und die an den Korb anzubringen sind. Durch ein Rahmenelement, das aus Draht gebogen ist, entfallen weiterhin scharfe Kanten, die zu Verletzungen führen können.

Vorteilhafterweise kann erfindungsgemäß vorgesehen sein, dass die Haken jeweils aus einem Drahtabschnitt gebildet werden, der zwei weitgehend parallel zueinander, parallel zu dem Boden verlaufende Abschnitte aufweist, wobei die dem Korb abgewandten Bereiche der Abschnitte durch einen gebogenen, weitgehend halbkreisförmigen Drahtabschnitt miteinander verbunden werden und wobei die halbkreisförmigen Drahtabschnitte vorzugsweise in einer zu dem Boden weitgehend senkrecht verlaufenden Ebene liegen. Eine derartige Ausgestaltung der Haken kann auf einfache Art und Weise realisiert werden und führt zu einem funtkionssicheren Stapeln zweier erfindungsgemäßer Körbe.

Weiterhin ist denkbar, dass sich die halbkreisförmigen Drahtabschnitte in Richtung der Oberkante der Seitenwände erstrecken. Dies hat den Vorteil, dass der erfindungsgemäße Korb auf dem Bodenrahmenelement satt zum Aufliegen kommen kann und ein "Wackeln" des Korbes auf einer ebenen Fläche, beispielsweise einem Untergrund, ausgeschlossen wird.

Nach einer Ausgestaltung der Erfindung kommen die Innenseiten der halbkreisförmigen Drahtabschnittes des oberen Korbes bei aufeinander gestapelten Körben auf den Ösen des unteren Korbes zum Aufliegen. Durch ein derartiges Eingreifen der Ösen in die Haken wird der obere Korb funktionssicher auf dem unteren Korb gestapelt. Über das Zusammenwirken von Ösen und Haken können insbesondere auch auf den oberen Korb wirkende horizontal angreifende Kräfte sicher in den unteren Korb abgeleitet werden.

Eine weitere Ausführungsform der Erfindung sieht vor, dass der Winkel zwischen den Seitenwänden und dem Boden größer als 90° und vorzugsweise etwa 100° bis 120° beträgt. Die genannten Werte haben sich hierbei als besonders vorteilhaft erwiesen, wobei ein günstiges Ineinanderstapelung von mehreren Körben erreichbar ist.

Erfindungsgemäß kann vorgesehen sein, dass an den Seitenwänden ein weitgehend parallel zu dem Boden verlaufendes Mittelrahmenelement vorhanden ist. Ein derartiges Mittelrahmenelement stabilisiert in erster Linie die Körbe.

Vorteilhafterweise kann erfindungsgemäß weiterhin vorgesehen sein, dass wenigstens zwei Körbe derart ineinander stapelbar sind, dass das Mittelrahmenelement des oberen Korbes auf der Oberseite der Ösen des unteren Korbes zum Aufliegen kommt.

Alternativ ist denkbar, dass die Unterseiten der Haken des oberen Korbes auf der Oberseite des Mittelrahmenelements des unteren Korbes zum Aufliegen kommt.

Vorzugsweise ist der Korb aus Edelstahl ausgeführt, da Edelstahl gegenüber dem Reinigungs- und/oder Sterilisationsbad besonders resistent ist.

Weitere vorteilhafte Ausgestaltungen und Einzelheiten der Erfindung sind der folgenden Beschreibung zu entnehmen, in der die Erfindung anhand der in der Zeichnung dargestellten Ausführungsbeispiele näher beschrieben und erläutert ist. Es zeigen:
- Figur 1: einen erfindungsgemäßen Korb in perspektivischer Ansicht;
- Figur 2: die Rahmenelemente samt Mittel- und Bodenrahmenelements des Korbes gemäß Fig. 1 in perspektivischer Ansicht;
- Figur 3 ohne: eine Draufsicht auf den Korb gemäß Figur 1 Drahtgitter;
- Figur 4: die Seitenansicht zweier aufeinander gestapelter Körbe;
- Figur 5: die perspektivische Ansicht einer Ösen mit einem mit der Öse zusammenwirkenden Haken;
- Figur 6: einen Schnitt entlang der Linie VI gemäß Figur 3 durch den in Figur 1 dargestellten Korb mit einem weiteren daraufgestapelten erfindungsgemäßen Korb; und
- Figur 7: einen der Figur 6 entsprechenden Schnitt zweier ineinander gestapelter Körbe.

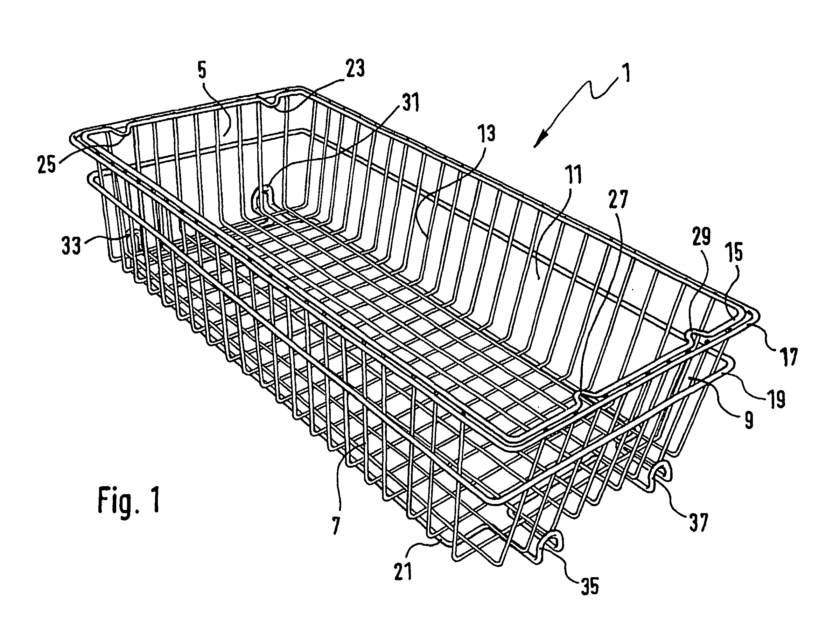

Figur 1 zeigt einen Korb 1, der als Sterilisations-, Transport- und auch als Lagerkorb für entsprechende Güter Verwendung finden kann. Der Korb 1 weist einen Boden 3 und vier Seitenwände 5, 7, 9 und 11. Der Boden 3 und die Seitenwände 5,7,9 und 11 werden hierbei von einem aus einzelnen, miteinander verschweißten Drähten bestehenden Drahtgitter 13 gebildet. Das Drahtgitter 13 des Korbes 1 ist relativ großmaschig ausgebildet. Erfindungsgemäß kann allerdings vorgesehen sein, dass das Drahtgitter 13 auch sehr feinmaschig ist, und zwar insbesondere dann, wenn relativ kleine Güter im dem Korb 1 sterilisiert, transportiert oder auch gelagert werden sollen.

Der Korb 1 weist an den Oberkanten der Seitenwände 5 bis 11 zwei weitgehend parallel nebeneinander verlaufende Rahmenelemente 15 und 17 aus einem geschlossenen Draht ohne freie Enden auf. Dadurch, dass die freien Enden der das Drahtgitter 13 bildenden Drähte zwischen den beiden Rahmenelementen 15 und 17 enden, ist eine Verletzungsgefahr an den freien Enden der entsprechenden Drähte ausgeschlossen.

Der Korb 1 weist außerdem zur Stabilisierung ein Mittelrahmenelement 19 aus einem geschlossenen Draht ohne freie Enden auf, das die Seitenwände 5 bis 11 umläuft, und weitgehehend parallel zu den beiden Rahmenelementen 15 und 17 auf der Korbaußenseite an den Seitenwänden 5 bis 11 angeordnet ist.

An der Unterseite weist der Korb 1 ein Bodenrahmenelement 21 auf, das ebenfalls aus einem entsprechend geformten Draht gebildet ist.

Wie aus Figur 1 deutlich hervorgeht, sind im Bereich der Oberkanten der Seitenwände 5 und 9 vier Ösen 23, 25, 27 und 29 vorhanden. Im Bereich der Unterkanten der Seitenwände 5 und 9 sind vier Haken 31, 33, 35 und 37 zu erkennen.

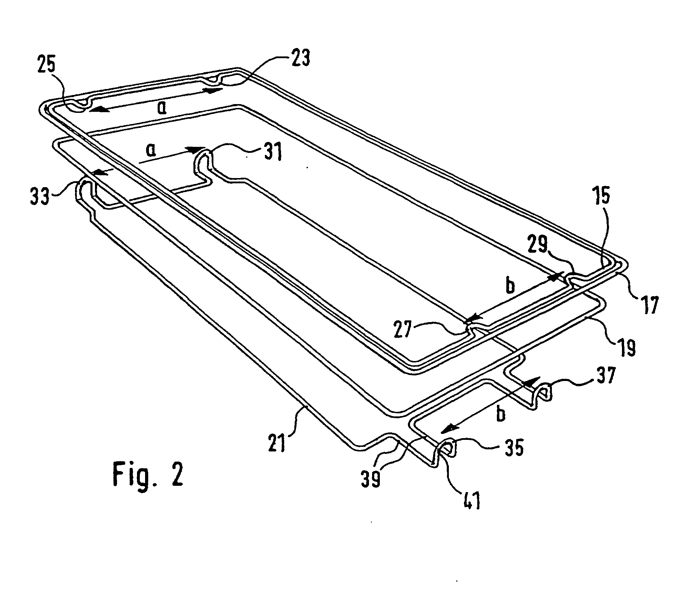

Figur 2 zeigt die beiden Rahmenelemente 15 und 17, das Mittelrahmenelement 19 und das Bodenrahmenelement 21 des Korbes 1 ohne das Drahtgitter 13. Hierbei ist deutlich zu erkennen, dass die Ösen 23 bis 29 von Verformungen des innenliegenden Rahmenelements 15 gebildet werden. Die Ösen 23 bis 29 liegen hierbei in der von den beiden Rahmenelementen 15 und 17 gebildeten Ebene und ragen in Richtung der Korbinnenseite.

Die Ösen 23 und 25, die an der Oberkante der Seitenwand 5 angeordnet sind, weisen einen größeren Abstand a als die beiden an der Oberkante der Seitenwand 9 angeordneten Ösen 27 und 29 auf, deren Abstand b beträgt.

Aus Figur 2 ist deutlich zu erkennen, dass die Haken 31, 33, 35 und 37 von Verformungen des Bodenrahmenelements 21 gebildet werden. Die einzelnen Haken 31 bis 37 werden hierbei von einem Drahtabschnitt gebildet, der zwei parallel zueinander, in einer zu dem Boden 3 parallel verlaufende Abschnitte 39 aufweist, wobei die dem Korb 1 abgewandten Bereiche der Abschnitte 39 durch einen gebogenen, weitgehend halbkreisförmigen Drahtabschnitt 41 miteinander verbunden werden. Der halbkreisförmige Drahtabschnitt 41 liegt hierbei vorzugsweise in einer zu dem Boden 3 senkrecht verlaufenden Ebene und ragt in Richtung der Korboberkante.

Die im Bereich der Unterkante der Seitenwand 5 vorhandenen Haken 31 und 35 weisen, entsprechend den an der gleichen Seitenwand 5 vorhandenen Ösen 23 und 25, den Abstand a zueinander auf. Die beiden Haken 35 und 37 der gegenüberliegenden Seitenwand 7 weisen entsprechend den beiden Ösen 27 und 29 den Abstand b zueinander auf, wobei b kleiner als a ist.

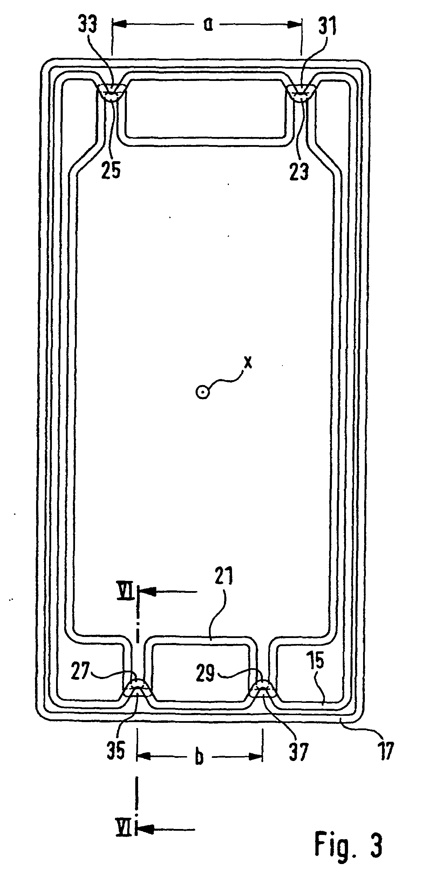

Der Figur 3, welche den Korb 1 ohne das Drahtgitter 13 in Draufsicht zeigt, sind die Maße a und b deutlich zu entnehmen.

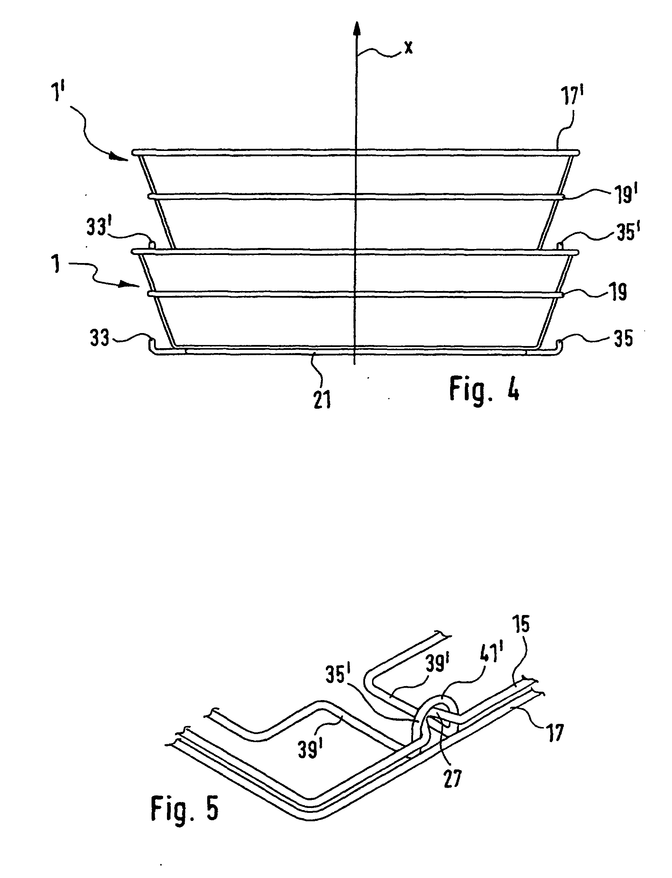

Figur 4 zeigt einen auf den Korb 1 gestapelten Korb 1' in Seitenansicht. Die dem Korb 1 entsprechenden Abschnitte und Merkmale des Korbes 1' sind im Folgenden mit " ' " gekennzeichnet.

Aufgrund der Anordnung der Ösen 23 bis 29 und der Haken 31 bis 37 können zwei erfindungsgemäße Körbe 1 und 1' derart aufeinander gestapelt werden, dass die Haken 31', 33', 35' und 37' des auf den Korb 1 stapelbaren Korbes 1' an den Ösen 23, 25, 27 und 29 des Korbes 1 zum Aufliegen kommen. Hierbei kommen die Innenseiten der halbkreisförmigen Drahtabschnitte 41' auf den jeweiligen Ösen 23 bis 29 zum Aufliegen.

Figur 5 zeigt die beiden Rahmenelemente 15 und 17 des unteren Korbes 1 mit der Öse 27 gemäß Figur 2 in vergrößerter Darstellung. Außerdem ist der Haken 35' des oberen Korbes 1' deutlich zu erkennen, dessen Innnenseite an der Oberseite der Öse 27 aufliegt.

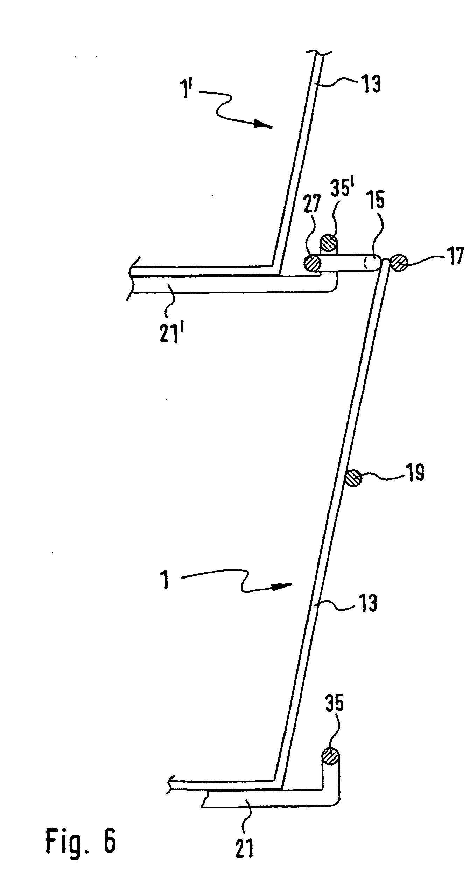

Bei dem in Figur 6 gezeigten Ausschnitt der beiden aufeinander gestapelten Körbe 1 und 1' ist im dargestellten Schnitt deutlich zu erkennen, wie die Innenseite des Hakens 35' an der Außenseite der Öse 27 anliegt.

Aufgrund der entsprechenden Anordnung der Ösen 23 bis 27 und der Haken 31 bis 37 ist es möglich, zwei oder mehrere Körbe 1 auf einfache Art und Weise aufeinander zu stapeln.

Dadurch, dass der Abstand a größer als der Abstand b ist, können mehrere Körbe 1 und 1' auch ineinander gestapelt werden. Hierbei wird einer der beiden Körbe 1 oder 1' um die senkrechte Mittelachse x um 180° gedreht, wobei dann beim Ineinandersetzen die Haken 31', 33', 35' und 37' des oberen Korbes 1' nicht an den Ösen 23, 25, 27 und 29 des unteren Korbes 1 zum Aufliegen kommen. Vielmehr liegen hierbei die beiden Haken 35 und 37, die den Abstand b aufweisen, zwischen den beiden weiter entfernt voneinander gelegenen Ösen 23 und 25. Entsprechend sind die beiden Haken 31 und 33, die den Abstand a zueinander aufweisen, rechts und links neben den beiden mit dem kleineren Abstand b voneinander entfernten Ösen 27 und 29 angeordnet. Damit können folglich zwei oder mehrere Körbe 1 und 1' ineinander gestapelt werden.

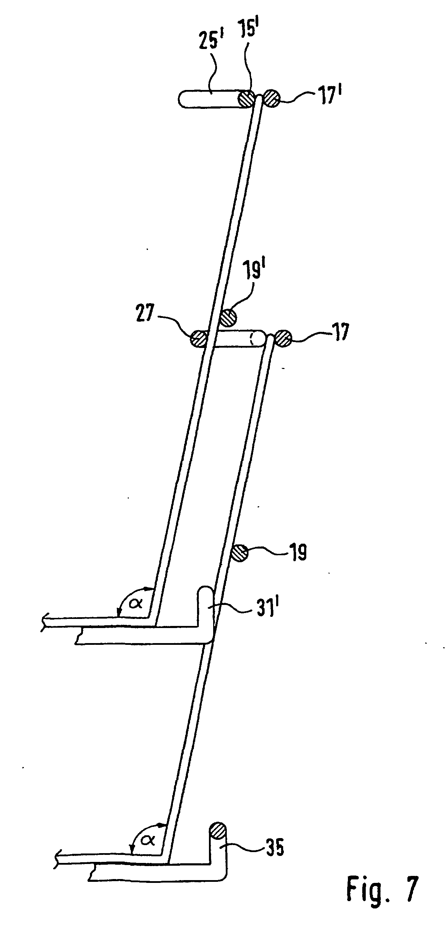

Figur 7 zeigt einen Schnitt durch zwei derartig ineinander gestapelte Körbe 1 und 1'. Da der Haken 31 nicht an der Öse 27 zum Aufliegen kommt, liegt dieser innerhalb des Korbes 1. Hierbei ist vorgesehen, dass die Haken 31', 33', 35' und 37' des oberen Korbes 1' an dem Mittelrahmenelement 19 des unteren Korbes 1 zum Aufliegen kommen. Damit kann ein definiertes und sicheres Aufeinanderstapeln von ein oder mehreren Körben 1 und 1' erreicht werden.

Die in den Figuren 1 bis 7 dargestellten Körbe 1 und 1' sind vorteilhafterweise aus Edelstahl.

Alle in der Beschreibung, den nachfolgenden Ansprüchen und der Zeichnung dargestellten Merkmale können sowohl einzeln als auch in beliebiger Kombination miteinander erfindungswesentlich sein.

## Patentansprüche

1. Korb (1, 1'), insbesondere Sterilisations-, Transportund/oder Lagerkorb, mit einem Boden (3) und mit Seitenwänden (5, 7, 9, 11), wobei im Bereich der Oberkanten der Seitenwände (5, 9) Ösen (23, 25, 27, 29) und im Bereich der Unterkanten der Seitenwände (5, 9) Haken (31, 33, 35, 37) vorhanden sind, wobei zwei identische Körbe (1, 1') derart aufeinander stapelbar sind, dass die Haken (31', 33', 35', 37') des oberen Korbes (1') an oder in den Ösen (23, 25, 27, 29) des unteren Korbes (1) zum Aufliegen kommen, **dadurch gekennzeichnet, dass** nach dem Drehen des oberen Korbes (1') um die senkrechte Mittelachse (x) um 180° die Haken (31', 33', 35', 37') des oberen Korbes (1') nicht an oder in den Ösen (23, 25, 27, 29) des unteren Korbes (1) zum Aufliegen kommen und die Körbe (1, 1') ineinander stapelbar sind.

2. Korb (1, 1') nach Anspruch 1, **dadurch gekennzeichnet,**
**dass** der Korb (1, 1') im Bereich der Oberkanten von zwei gegenüberliegenden Seitenwänden (5, 9) jeweils zwei Ösen (23, 25 oder 27, 29) aufweist, wobei der Abstand (a) der beiden Ösen (23, 25) zueinander an der einen Seitenwand (5) größer als der Abstand (b) der beiden Ösen (27, 29) an der gegenüberliegenden Seitenwand (9) ist, und dass der Korb (1, 1') im Bereich der Unterkanten der entsprechenden Seitenwänden (5, 9) jeweils zwei Haken (31, 33 oder 35, 37) aufweist, wobei der Abstand (a) der beiden Haken (31, 33) zueinander an der einen Seitenwand (5) größer ist als der Abstand (b) der beiden Haken (35, 37) an der gegenüberliegenden Seitenwand (9) und wobei die Abstände (a oder b) der Haken (31, 33 oder 35, 37) zueinander den Abständen (a oder b) der Ösen (23, 25 oder 27, 29) zueinander der gleichen Seitenwand (5 oder 9) entsprechen.

3. Korb (1, 1') nach einem der vorhergehenden Ansprüche, **dadurch gekennzeichnet, dass** die Ösen (23, 25, 27, 29) der Korbinnenseite zugewandt sind.

4. Korb (1, 1') nach einem der vorhergehenden Ansprüche, **dadurch gekennzeichnet, dass** die Haken (31, 33, 35, 37) den Oberkanten der Seitenwände (5, 9) zu- oder abgewandt sind.

5. Korb (1, 1') nach einem der vorhergehenden Ansprüche, **dadurch gekennzeichnet, dass** der Korb (1, 1') an der Oberkante der Seitenwände (5, 7, 9, 11) zwei parallel nebeneinander verlaufende Rahmenelemente (15, 17) aus Draht aufweist, wobei die Ösen (23, 25, 27, 29) von Verformungen des innen liegenden Rahmenelements (15) gebildet werden.

6. Korb (1, 1') nach Anspruch 5, **dadurch gekennzeichnet, dass** der Boden (3) und die Seitenwände (5, 7, 9, 11) von einem zusammenhängenden Drahtgitter (13) gebildet werden, wobei freie Enden der das Drahtgitter (13) bildenden Drähte zwischen den beiden Rahmenelementen (15, 17) enden.

7. Korb (1, 1') nach einem der vorhergehenden Ansprüche, **dadurch gekennzeichnet, dass** der Korb (1, 1') an der Unterkante der Seitenwände (5, 7, 9, 11) ein Bodenrahmenelement (21) aus Draht aufweist, wobei die Haken (31, 33, 35, 37) von Verformungen des Bodenrahmenelements (21) gebildet werden.

8. Korb (1, 1') nach einem der vorhergehenden Ansprüche, **dadurch gekennzeichnet, dass** die Haken (31, 33, 35, 37) jeweils aus einem Drahtabschnitt gebildet werden, der zwei parallel zueinander, parallel zu dem Boden verlaufende Abschnitte (39) aufweist, wobei die dem Korb (1, 1') abgewandten Bereiche der Abschnitte (39) durch einen gebogenen, halbkreisförmigen Drahtabschnitt (41) miteinander verbunden werden und wobei die halbkreisförmigen Drahtabschnitte (41) in einer zu dem Boden (3) senkrecht verlaufenden Ebene liegen.

9. Korb (1, 1') nach Anspruch 8, **dadurch gekennzeichnet, dass** sich die halbkreisförmigen Drahtabschnitte (41) in Richtung der Oberkanten der Seitenwände (5, 9) erstrecken.

10. Korb (1, 1') nach Anspruch 8 oder 9, **dadurch gekennzeichnet, dass** die Innenseiten der halbkreisförmigen Drahtabschnitte (41') des oberen Korbes (1') bei aufeinander gestapelten Körben (1, 1') auf den Ösen (23, 25, 27, 29) des unteren Korbes (1) zum Aufliegen kommen.

11. Korb (1, 1') nach einem der vorhergehenden Ansprüche, **dadurch gekennzeichnet, dass** der Winkel (á) zwischen den Seitenwänden und dem Boden (3) größer als 90° und vorzugsweise etwa 100° - 120° beträgt.

12. Korb (1, 1') nach einem der vorhergehenen Ansprüche, **dadurch gekennzeichnet, dass** an den Seitenwänden (5, 7, 9, 11) ein parallel zu dem Boden verlaufendes Mittelrahmenelement (19) vorhanden ist.

13. Korb (1, 1') nach Anspruch 12, **dadurch gekennzeichnet, dass** wenigstens zwei Körbe (1, 1') derart ineinander stapelbar sind, dass das Mittelrahmenelement (19') des oberen Korbes (1') auf der Oberseite der Ösen (23, 25, 27, 29) des unteren Korbes (1) zum Aufliegen kommt.

14. Korb (1, 1') nach einem der vorhergehenden Ansprüche, **dadurch gekennzeichnet, dass** der Korb (1, 1') vollständig aus Edelstahl ist.

## Claims

1. A basket (1,1'), in particular a sterilization, transport, and/or storage basket, comprising a base (3) and side walls (5, 7, 9, 11), eyes (23, 25, 27, 29) being present in the area of the upper edge of the side walls (5, 9), and hooks (31, 33, 35, 37) being present in the area of the lower edge of the side walls (5, 9), it being possible to stack two identical baskets (1, 1') on top of each other such that the hooks (31', 33', 35', 37') of the upper basket (1') rest on or in the eyes (23, 25, 27, 29) of the lower basket (1), wherein, after the upper basket (1') is rotated about its vertical central axis (x) by 180°, the hooks (31', 33', 35', 37') of the upper basket (1') do not rest on or in the eyes (23, 25, 27, 29) of the lower basket (1), and the baskets (1, 1') can be stacked one inside the other.

2. The basket (1, 1') as recited in Claim 1, wherein the basket (1, 1') in the area of the upper edges of two opposite side walls (5, 9) has, in each case, two eyes (23, 25 or 27, 29), the distance (a) between the two eyes (23, 25) on one side wall (5) being greater than the distance (b) between the two eyes (27, 29) on the opposite side wall (9), and the basket (1, 1') in the area of the lower edges of the corresponding side walls (5, 9) having, in each case, two hooks (31, 33 or 35, 37), the distance (a) between the two hooks (31, 33) on one side wall (5) being greater than the distance (b) between the two hooks (35, 37) on the opposite side wall (9), and the distance (a or b) between the hooks (31, 33 or 35, 37) corresponding to the distance (a or b) between the eyes (23, 25 or 27, 29) on the same side wall (5 or 9).

3. The basket (1, 1') as recited in any of the preceding claims, wherein the eyes (23, 25, 27, 29) face the interior of the basket.

4. The basket (1, 1') as recited in any of the preceding claims, wherein the hooks (31, 33, 35, 37) are facing toward or away from the upper edge of the side walls (5, 9).

5. The basket (1, 1') as recited in any of the preceding claims, wherein the basket (1, 1') on the upper edge of the side walls (5, 7, 9, 11) has two wire frame elements (15, 17) that are adjacent and parallel to each other, the eyes (23, 25, 27, 29) being constituted by deformations of the inner frame element (15).

6. The basket (1, 1') as recited in Claim 5, wherein the base (3) and the side walls (5, 7, 9, 11) are constituted by a continuous wire grating (13), the free ends of wires that make up the wire grating (13) terminating between the two frame elements (15, 17).

7. The basket (1, 1') as recited in any of the preceding claims, wherein the basket (1, 1') on the lower edge of the side walls (5, 7, 9, 11) has a base frame element (21) made of wire, the hooks (31, 33, 35, 37) being constituted by deformations of the base frame element (21).

8. The basket (1, 1') as recited in any of the preceding claims, wherein each hook (31, 33, 35, 37) is made up of a wire segment that has two segments (39) that are parallel to each other and parallel to the base, the areas of the segments (39) that face away from the basket (1, 1') being joined to each other by a bent, semicircular wire segment (41), and the semicircular wire segments (41) being situated in a plane that is perpendicular to the base (3).

9. The basket (1, 1') as recited in Claim 8, wherein the semicircular wire segments (41) extend in the direction of the upper edge of the side walls (5, 9).

10. The basket (1, 1') as recited in Claim 8 or 9, wherein, when the baskets (1, 1') are stacked one on top of the other, the interior side of the semicircular wire segments (41') of the upper basket (1') rests on the eyes (23, 25, 27, 29) of the lower basket (1).

11. The basket (1, 1') as recited in any of the preceding claims, wherein the angle (á) between the side walls and the base (3) is greater than 90° and preferably between 100° and 120°.

12. The basket (1, 1') as recited in any of the preceding claims, wherein a central frame element (19) that is parallel to the base is present on the side walls (5, 7, 9, 11).

13. The basket (1, 1') as recited in Claim 12, wherein at least two baskets (1, 1') can be stacked inside each other such that the central frame element (19') of the upper basket (1') rests on top of the upper side of the eyes (23, 25, 27, 29) of the lower basket (1).

14. The basket (1, 1') as recited in any of the preceding claims, wherein the basket (1, 1') is entirely made of special steel.

## Revendications

1. Panier (1, 1'), plus particulièrement panier de stérilisation, transport et/ou de stockage avec un fond (3) et avec des parois latérales (5, 7, 9 11), moyennant quoi au niveau des arêtes supérieures des parois latérales (5, 9) se trouvent des oeillets (23, 25, 27, 29) et, au niveau des arêtes inférieures des parois latérales (5, 9), se trouvent des crochets 31, 33, 35, 37), moyennant quoi deux paniers identiques (1, 1') peuvent être empilés l'un sur l'autre de telle sorte que les crochets (31', 33', 35', 37') du panier supérieur (1') s'appuient sur ou dans les oeillets (23, 25, 27, 29) du panier inférieur (1), **caractérisé en ce qu'**après la rotation du panier supérieur (1') autour de l'axe central vertical (x) de 180°, les crochets (31', 33', 35', 37') du panier supérieur (1') ne s'appuient pas sur ou dans les oeillets (23, 25, 27, 29) du panier inférieur (1) et les paniers (1, 1') sont empilables l'un,dans l'autre.

2. Panier (1, 1') selon la revendication 1, **caractérisé en ce que** le panier (1, 1') comporte, au niveau des arêtes supérieures de deux parois latérales opposées (5, 9) deux oeillets (23, 25 ou 27, 29), moyennant quoi la distance (a) entre les deux oeillets (23, 25) est supérieur, au niveau d'une paroi latérale (5), à la distance (b) entre les deux oeillets (27, 29) au niveau de la paroi latérale opposée (9), et **en ce que** le panier (1, 1') comporte, au niveau des arêtes inférieures des parois latérales (5, 9) correspondantes, deux oeillets (31, 33 ou 35, 37), moyennant quoi la distance (a) entre les deux oeillets (31, 33) est supérieur, au niveau d'une paroi latérale (5), à la distance (b) entre les deux crochets (35, 37) au niveau de la paroi latérale opposée (9) et moyennant quoi les distances (a ou b) entre les crochets (31, 33 ou 35, 37) correspondent aux distances (a ou b) entre les oeillets (23, 25 ou 27, 29) de la même paroi latérale (5 ou 9).

3. Panier (1, 1') selon l'une des revendications précédentes, **caractérisé en ce que** les oeillets (23, 25, 27, 29) sont orientés vers l'intérieur du panier.

4. Panier (1, 1') selon l'une des revendications précédentes, **caractérisé en ce que** les crochets (31, 33, 35, 37) sont orientés vers ou du côté opposé aux arêtes supérieures des parois latérales (5, 9).

5. Panier (1, 1') selon l'une des revendications précédentes, **caractérisé en ce que** le panier (1, 1') comporte, au niveau de l'arête supérieure des parois latérales (5, 7, 9, 11), deux éléments d'encadrement parallèles (15, 17) en fil, moyennant quoi les oeillets (23, 25, 27, 29) sont constitués par des déformations intérieures de l'élément d'encadrement (15).

6. Panier (1, 1') selon la revendication 5, **caractérisé en ce que** le fond (3) et les parois latérales (5, 7, 9, 11) sont constitués d'une grille (13), moyennant quoi les extrémités libres des fils constituant la grille (13) se terminent entre les deux élément d'encadrement (15, 17).

7. Panier (1, 1') selon l'une des revendications précédentes, **caractérisé en ce que** le panier (1, 1') comporte, au niveau de l'arête inférieur des parois latérales (5, 7, 9, 11), un élément d'encadrement de fond (21) en fil, moyennant quoi les crochets (31, 33, 35, 37) sont constitués par des déformations de l'élément d'encadrement du fond (21).

8. Panier (1, 1') selon l'une des revendications précédentes, **caractérisé en ce que** les crochets (31, 33, 35, 37) sont constitués d'une partie en fil qui comporte deux parties (39) parallèles entre elles et parallèles au fond, moyennant quoi les zones des parties (39) orientées du côté opposé au panier (1, 1') sont reliées entre elles par une partie en fil (41) pliée en forme de demi-cercle et moyennant quoi les parties en fil (41) en forme de demi-cercle se trouvent dans un plan perpendiculaire au fond (3).

9. Panier (1, 1') selon la revendication 8, **caractérisé en ce que** les parties en fil (41) semi-circulaires s'étendent en direction des arêtes supérieures des parois latérales (5, 9).

10. Panier (1, 1') selon la revendication 8 ou 9, **caractérisé en ce que** les côtés internes des parties en fil (41') semi-circulaires du panier supérieur (1') s'appuient sur les oeillets (23, 25, 27, 29) du panier inférieur (1) lorsque les paniers (1, 1') sont empilés l'un sur l'autre.

11. Panier (1, 1') selon l'une des revendications précédentes, **caractérisé en ce que** l'angle (a) entre les parois latérales et le fond (3) est supérieur à 90° et de préférence d'environ 100° à 120°.

12. Panier (1, 1') selon l'une des revendications précédentes, **caractérisé en ce qu'**au niveau des parois latérales (5, 7, 9, 11), se trouve un élément d'encadrement central (19) parallèle au fond.

13. Panier (1, 1') selon la revendication 12, **caractérisé en ce qu'**au moins deux paniers (1, 1') sont empilables l'un dans l'autre de telle sorte que l'élément d'encadrement central (19') du panier supérieur (1') s'appuie sur le côté supérieur des oeillets (23, 25, 27, 29) du panier inférieur (1).

14. Panier (1, 1') selon l'une des revendications précédentes, **caractérisé en ce que** le panier (1, 1') est constitué entièrement d'acier inoxydable.
